# EUROPÄISCHE PATENTSCHRIFT

(11) **EP 1 200 150 B1**
(45) Veröffentlichungstag und Bekanntmachung des Hinweises auf die Patenterteilung: **03.03.2004**
(21) Anmeldenummer: 99944339.3
(22) Anmeldetag: 06.08.1999
(51) Int. Cl.: A61M 16/04

(54) **TRACHEALKANÜLE**
TRACHEAL CANNULA
CANULE TRACHEALE

(43) Veröffentlichungstag der Anmeldung: 02.05.2002
(73) Patentinhaber: Zylka-Eistert, Maria, 91541 Rothenburg ob der Tauber (DE)
(72) Erfinder: EISTERT, Bernhard, D-91541 Rothenburg ob der Tauber (DE)
(74) Vertreter: KEIL & SCHAAFHAUSEN
(86) Internationale Anmeldenummer: PCT/EP1999/005696
(87) Internationale Veröffentlichungsnummer: WO 2001/010490

(56) Entgegenhaltungen:
- EP-A- 0 106 780
- WO-A-99/32169
- DE-C- 19 803 572
- DE-U- 29 613 819
- US-A- 5 771 888

## Beschreibung

Die Erfindung betrifft eine Trachealkanüle zum Einsetzen in die Luftröhre nach einem Luftröhrenschnitt mit einem Schaft und einer Manschette zum Blockieren des den Schaft umgebenden Luftröhrenquerschnitts.

Nach einem Luftröhrenschnitt (Tracheotomie) wird eine Kanüle in die Luftröhre eingesetzt, um das geschaffene Lumen offenzuhalten. Hierzu stehen Kanülen in verschiedenartiger Form und aus unterschiedlichen Materialien zur Verfügung. So sind dünnwandige Silberkanülen bekannt, die doppelläufig, d.h. mit einer Außen- und einer leicht zu entfernenden Innenkanüle ausgebildet sind. Ferner gibt es dickwandigere Kunststoffkanülen, die im Gegensatz zu Metallkanülen nicht so steif sind und sich bei Körpertemperatur etwas der Form der Umgebung anpassen, ohne ihr Lumen dabei zu verändern.

Besteht eine erhöhte Aspirationsgefahr, so wird eine Trachealkanüle mit einer sog. Blockermanschette verwendet, über die ein Eindringen von Speichel, Magensaft und anderen Flüssigkeiten oder Nahrung (Aspiration) verhindert werden soll. Diese Manschetten sind üblicherweise um den Schaft der Kanüle angeordnet oder in diesen integriert und aufblasbar, um sich der Luftröhrenumgebung anzupassen.

Schließlich gibt es Sprechkanülen, die mit einem Ventilmechanismus und einem Loch oder einem Sieb an ihrem Schaft ausgestattet sind, um dem Patienten das Sprechen zu ermöglichen. Bei der Inspiration öffnet sich der Ventilmechanismus am Kanüleneingang und ermöglicht die Zufuhr von Luft durch die Kanüle in die Lunge. Bei der Ausatmung verschließt sich dagegen das Ventil und der Luftstrom wird durch das Kanülenloch oder -fenster auf normalem Wege durch den Kehlkopf herausgeführt, so daß sowohl eine normale Luftzufuhr als auch der Gebrauch der Stimme ermöglicht wird. Derartige Sprechkanülen sind jedoch nur bei nicht aspirationsgefährdeten Patienten einsetzbar, so daß diese keine Möglichkeit haben zu sprechen. Während dies im Übergangsstadium unmittelbar nach der Tracheotomie für die Patienten noch tolerabel ist, ist die dauerhafte Sprechverhinderung insbesondere bei neurologischen Patienten mit langandauernder Aspirationsgefahr (beispielsweise nach einem Schlaganfall) nur schwer hinnehmbar.

Aus der US 4 459 984 ist eine gattungsgemäße Trachealkanüle zum Einsetzen in die Luftröhre bekannt mit einer gekrümmten Kanüle, um deren in der Luftröhre befindliches Ende eine aufblasbare Manschette angeordnet ist. Die Trachealkanüle weist oberhalb der Manschette eine mit einem Klappenventil verschließbare Austrittsdüse oder -öffnung auf, wobei das Ventil durch Erhöhung des Luftdrucks in der Trachealkanüle öffenbar ist, so dass Luft in die obere Speiseröhre austritt und der Patient während der mechanischen Entlüftung sprechen kann. Durch das Ventil, welches im geschlossenen Zustand eine Aspiration verhindern soll, wird der Luftstrom allerdings stark umgelenkt und verlangsamt. Dadurch wird das natürliche Sprechverhalten im Kehlkopf beeinträchtigt. Ferner wird das Ventil bei nachlassendem Innendruck in der Trachealkanüle abrupt geschlossen und verhindert somit das Weitersprechen. Außerdem kann durch ein mögliches Verklemmen der Klappe des Ventils eine Aspirationsgefahr nicht zuverlässig ausgeschlossen werden.

Aufgabe der Erfindung ist es daher, auch bei Trachealkanülen mit Manschette zuverlässig ein möglichst natürliches Sprechen zu ermöglichen.

Diese Aufgabe wird mit der Erfindung im wesentlichen dadurch gelöst, daß in dem oberhalb der Manschette liegenden Abschnitt des Schaftes ein Fenster ausgebildet ist, das durch eine luftdurchlässige Membran abgedeckt ist. Die Membran verhindert somit das Eindringen von Speichel und Speisen in die Kanüle und damit die unteren Luftwege. Zum anderen wird aber aufgrund der Luftdurchlässigkeit der Membran das Sprechen ermöglicht.

Gemäß einer bevorzugten Ausgestaltung der Erfindung ist die Membran nicht wasserdurchlässig, um zu gewährleisten, daß keinerlei Speichel- oder Nahrungsbestandteile in die Kanüle eintreten. Es hat sich hierbei als besonders vorteilhaft herausgestellt, eine Membran aus Polytetrafluorethylen (PTFE) zu verwenden, insbesondere ein Gewebe aus PTFE, wie es unter dem Handelsnamen Gore-Tex® im geschäftlichen Verkehr erhältlich ist.

Beim Einatmen wird die Luft durch die Kanüle in die Lunge geführt, während der Patient zum Sprechen die Kanüle zuhalten kann, so daß die Luft durch den Membran in den Kehlkopf gelangt. Gemäß einer bevorzugten Ausgestaltung der Erfindung ist jedoch vorgesehen, daß am Eingang der Kanüle ein Ventil vorgesehen ist, das sich beim Einatmen selbsttätig öffnet und beim Ausatmen schließt.

In Weiterbildung der Erfindung ist die Manschette über eine Leitung mit einem Pilotballon oder dergleichen zum Aufblasen der Manschette und zur Kontrolle des Manschettendruckes verbunden.

weitere Ziele, Vorteile und Anwendungsmöglichkeiten der Erfindung ergeben sich aus der nachfolgenden Beschreibung eines Ausführungsbeispiels und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen:

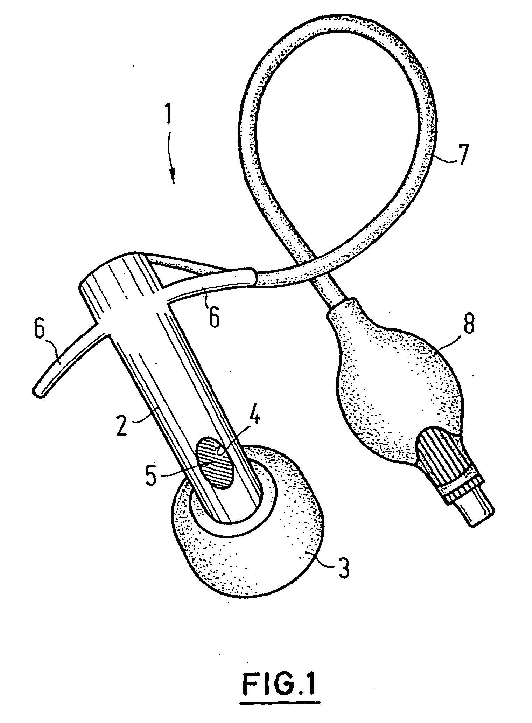

- Fig. 1: eine schematische Ansicht der erfindungsgemäßen Trachealkanüle und
- Fig. 2: schematisch die Trachealkanüle eingesetzt in die Luftröhre eines Patienten.

Wie aus der Zeichnung ersichtlich ist, besteht eine erfindungsgemäße Trachealkanüle 1 im wesentlichen aus einem hohlen Schaft 2, in dessen unterem Bereich eine Manschette 3 vorgesehen ist. Die Manschette 3 kann auf den Schaft 2 aufgeschoben oder einstückig mit diesem ausgebildet sein. Oberhalb der Manschette 3 ist in dem Schaft 2 ein Fenster 4 ausgebildet, das mit einer luftdurchlässigen aber nicht wasserdurchlässigen Membran 5 abgedeckt ist. Die Membran besteht vorzugsweise aus einem Gewebe, wie es unter dem Handelsnamen Gore-Tex® im geschäftlichen Verkehr erhältlich ist. Im vorderen Bereich des Schaftes 2 sind Haltearme 6 vorgesehen, über die die Position und Befestigung der Trachealkanüle 1 am Hals des Patienten erfolgen kann. Über eine Leitung 7, die mit einem Pilotballon 8 verbunden ist, kann die Manschette 3 aufgepumpt und der Manschettendruck kontrolliert werden. Die Leitung 7 kann abnehmbar an der Kanüle vorgesehen sein.

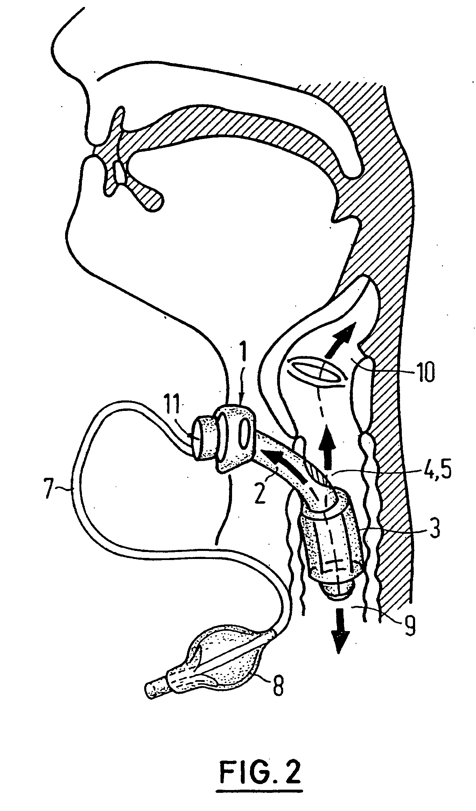

Die Trachealkanüle 1 wird nach einem Luftröhrenschnitt (Tracheotomie), wie in Fig. 2 dargestellt, in die Luftröhre 9 des Patienten eingesetzt. Hierbei ist darauf zu achten, daß das membrangeschützte Fenster 4 des Schaftes 2 im Tracheallumen der Luftröhre 9 liegt, damit beim Ausatmen die Luftführung in den Kehlkopf 10 gewährleistet ist. Nach dem Einsetzen der Trachealkanüle 1 wird die Manschette 3 mit Hilfe des Pilotballons 8 oder auf andere geeignete Weise aufgepumpt, so daß sie sich dem Luftröhrenquerschnitt anpaßt und die Luftröhre verschließt. Die Luftzufuhr ist damit allein über die Kanüle 1 möglich.

Beim Einatmen wird die Luft durch den Eingang 11 der Trachealkanüle 1 angesaugt und in die Lunge transportiert. Beim Ausatmen verschließt ein nicht dargestelltes Ventil die Kanüle 1, so daß der Luftstrom durch das membrangeschützte Fenster 4 in den Kehlkopf 10 austritt und der Patient sprechen kann.

Bei einer anderen, einfacheren Ausgestaltung ist in der Trachealkanüle 1 kein Ventil vorgesehen, so daß der Patient zum Sprechen die Kanüle 1 während der Expiration (Ausatmung) zuhalten muß.

Mit der Erfindung wird somit zuverlässig das Eintreten von Flüssigkeit in die Luftröhre verhindert, während beim Ausatmen Luft durch die Membran in den Kehlkopf gelangt, so daß der Patient sprechen kann.

### Bezugszeichenliste:

- 1: Trachealkanüle
- 2: Schaft
- 3: Manschette
- 4: Fenster
- 5: Membran
- 6: Haltearme
- 7: Leitung
- 8: Pilotballon
- 9: Luftröhre
- 10: Kehlkopf
- 11: Eingang

## Patentansprüche

1. Trachealkanüle zum Einsetzen in die Luftröhre (9) nach einem Luftröhrenschnitt mit einem Schaft (2) und einer Manschette (3) zum Blockieren des den Schaft (2) umgebenden Luftröhrenquerschnitts, daß in dem oberhalb der Manschette (3) liegenden Abschnitt des Schaftes (2) ein Fenster (4) ausgebildet ist, **dadurch gekennzeichnet, daß** durch eine luftdurchlässige Membran (5) abgedeckt ist.

2. Trachealkanüle nach Anspruch 1, **dadurch gekennzeichnet, daß** die Membran (5) nicht wasserdurchlässig ist.

3. Trachealkanüle nach Anspruch 1 oder 2, **dadurch gekennzeichnet, daß** die Membran (5) im wesentlichen aus Polytetrafluorethylen (PTFE) besteht.

4. Trachealkanüle nach Anspruch 3, **dadurch gekennzeichnet, daß** die Membran (5) aus einem Gewebe aus PTFE-Schnüren besteht.

5. Trachealkanüle nach einem der Ansprüche 1 bis 4, **dadurch gekennzeichnet, daß** am Eingang (11) der Kanüle (1) ein ventil vorgesehen ist, das sich beim Einatmen öffnet und beim Ausatmen schließt.

6. Trachealkanüle nach einem der Ansprüche 1 bis 5, **dadurch gekennzeichnet, daß** die Manschette (3) über eine Leitung (7) mit einem Pilotballon (8) oder dergleichen zum Aufblasen der Manschette (3) und zur Kontrolle des Manschettendrucks verbunden ist.

## Claims

1. Tracheal canula for insertion into the trachea (9) following a tracheotomy, having a shaft (2) and a cuff (3) for blocking the tracheal cross sectional area surrounding the shaft (2), wherein in the section of the shaft (2) lying above the cuff (3) a window (4) is constructed, **characterized in that** the window (4) is covered by an air permeable membrane (5).

2. Tracheal canula according to claim 1, **characterized in that** the membrane (5) is not permeable to water.

3. Tracheal canula according to claim 1 or 2, **characterized in that** the membrane (5) consists essentially of polytetrafluorethylene (PTFE).

4. Tracheal canula according to claim 3, **characterized in that** the membrane (5) consists of a fabric made of PTFE lacing.

5. Tracheal canula according to any of the claims 1 to 4, **characterized in that** at the entrance (11) of the canula (1) a valve is provided, which opens upon inhalation and closes upon exhalation.

6. Tracheal canula according to any of the claims 1 to 5, **characterized in that** the cuff (3) is connected via a line (7) to a pilot balloon (8) or the like for the inflation of the cuff (3) and for controlling the cuff pressure.

## Revendications

1. Canule trachéale pour l'insertion dans la trachée (9) après une trachéotomie avec une tige (2) et un manchon (3) pour bloquer la section transversale de la trachée entourant le tige (2), une fenêtre (4) étant formée dans la section de la tige (2) se situant au-dessus du manchon (3), **caractérisée en ce que** la fenêtre (4) est couverte par une membrane (5) perméable à l'air.

2. Canule trachéale selon la revendication 1, **caractérisée en ce que** la membrane (5) n'est pas perméable à l'eau.

3. Canule trachéale selon la revendication 1 ou 2, **caractérisée en ce que** la membrane (5) est essentiellement constituée de polytetrafluoréthylène (PTFE).

4. Canule trachéale selon la revendication 3, **caractérisée en ce que** la membrane (5) est constituée d'un tissu en cordon à PTFE.

5. Canule trachéale selon une des revendications 1 à 4, **caractérisée en ce qu**'une valve s'ouvrant lors de l'inspiration et se fermant lors de l'expiration est prévue à l'entrée (11) de la canule (1).

6. Canule trachéale selon une des revendications 1 à 5, **caractérisée en ce que** le manchon (3) est connecté à un ballon pilote (8) ou autre à travers un conduit (7) pour gonfler le manchon (3) et pour contrôler la pression du manchon.
